(19) Europäisches Patentamt
European Patent Office
Office européen des brevets

(11) **EP 4 560 310 A1**

(12) **EUROPEAN PATENT APPLICATION**
published in accordance with Art. 153(4) EPC

(43) Date of publication:
**28.05.2025 Bulletin 2025/22**

(21) Application number: **23843016.9**

(22) Date of filing: **19.07.2023**

(51) International Patent Classification (IPC):
***G01N 27/62*** (2021.01)

(52) Cooperative Patent Classification (CPC):
**G01N 27/62**

(86) International application number:
**PCT/JP2023/026460**

(87) International publication number:
**WO 2024/019093 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.07.2022   JP 2022114779
06.02.2023   JP 2023016166**

(71) Applicant: **NIPPON STEEL CORPORATION
Chiyoda-ku
Tokyo 100-8071 (JP)**

(72) Inventors:
• **TOBU, Yasuhiro
Tokyo 100-8071 (JP)**
• **AIMOTO, Michihiro
Tokyo 100-8071 (JP)**

(74) Representative: **Vossius & Partner
Patentanwälte Rechtsanwälte mbB
Siebertstrasse 3
81675 München (DE)**

(54) **QUANTITATIVE ANALYSIS METHOD AND QUANTITATIVE ANALYSIS DEVICE**

(57)     A quantitative analysis method and quantitative analysis device speeding detection and resistant to the effect of the condition of the device (fluctuations in sensitivity) in mass spectrometry using a mass spectrometer, i.e., a quantitative analysis method of a measured object using a mass spectrometer, which quantitative analysis method comprising a gas introduction step of mixing a gas of the measured object and a carrier gas different from the gas of the measured object and having two or more natural isotopes and introducing them to a mass spectrometer, a mass spectrometry step of mass spectrometry of the gas of the measured object and the two or more natural isotopes in the carrier gas at the mass spectrometer, and a gas concentration calculation step of calculating a concentration of the gas of the measured object using analyzed values of the two or more isotopes of the carrier gas obtained by the mass spectrometry step as calibration standards.

# Fig. 5

**Description**

FIELD

The present invention relates to a quantitative analysis method, in particular a quantitative analysis method for quantitatively analyzing a measured object using two or more natural isotopes contained in a carrier gas as standards, and to a quantitative analysis device.

BACKGROUND

Quantitative analysis is being utilized in various ways in the field of industry, the field of medical and life sciences, chemical analysis, etc. One of the typical quantitative analysis methods is mass spectrometry. The mass spectrometry method focuses on the mass of a substance covered, identifies the substance by a difference in mass, and quantifies it from the detection intensity. More particularly, a mass spectrometer is comprised of a sample introduction part, an ionization part (ion source), a mass separation part (analyzer), a detection part (detector), a vacuum exhaust part (vacuum pump), a device control/data processing part (data system), etc. At the time of measurement, the introduced sample is ionized by the ion source and rendered ions present in a gas phase. Ions differ in mobility depending on the mass to charge ratio (m/z), so various principles are used for separating them at the analyzer. A detector is used for detection. The analyzer and detector are maintained in a vacuum enabling a sufficient mean free path to be secured so that the ions do not strike other particles, and separation and detection are not impaired. It should be noted that, before introducing a sample into the mass spectrometer, the measured object may be segmented through chromatography. The mass spectrometry method has the merit, compared with other techniques, that it is possible to identify a large number of gas constituents with a high sensitivity by a single analysis. Further, it is possible to measure the concentration of a substance being measured by mass spectrometry.

The degree of vacuum has an extremely great impact on the sensitivity of detection in measurement in the mass spectrometry method. That is, sometimes the ionization efficiency in ionizing the substance covered, the drop in the number of ions due to unavoidable collision with other charges while in flight, and further the sensitivity of the detector itself detecting the charges are also affected by the degree of vacuum. For this reason, maintaining a certain extent of degree of vacuum is important for securing the condition of the device, the sensitivity of detection, and the quantifiability.

Each time starting up a mass spectrometer or even while starting it up, the degree of vacuum sometimes fluctuates, though only somewhat. For this reason, for quantitative analysis of a sample by a mass spectrometer, each time starting it up, the general practice is to prepare a standard sample and prepare a calibration curve using the same.

However, preparing such a standard sample involves some cost. Further, time is lost in preparing a calibration curve by a standard sample. In addition, depending on the degree of change of sensitivity of the mass spectrometer, it is necessary to redo the calculation curve very frequently and various other problems arise.

Up until now, various efforts have been made to raise the accuracy and reproducibility of mass spectrometry and increase work efficiency. For example, PLT 1 relates to a measurement method using mass spectrometry to enable real time measurement of the amount of unburned fuel in exhaust gas discharged from an internal combustion engine etc. Specifically, the amount of unburned gas in exhaust gas is calculated from the results of analysis at the time of measurement in mass spectrometry eliminating nonvolatile constituents in the exhaust gas to be measured and introducing to the mass spectrometer and analyzing only the volatile constituents in the exhaust gas and the results of prior analysis analyzing the samples of fuel producing the exhaust gas to be measured. It is described that by this method, the analysis equipment is simplified and measurement in an internal combustion engine mounted in an automobile etc. becomes possible while running.

PTL 2 relates to a high precision quantitative determination method using mass spectrometry. Specifically, it describes comprising

a step of calculating the quantitatively determined values of a detected substance based on calibration curves for each of several internal standard substances and averaging the quantitatively determined values to calculate a first quantitatively determined value,
a step of calculating a correction coefficient, and
a step of correcting the first quantitatively determined value by the correction coefficient to calculate a second quantitatively determined value,
the measurement for preparing calibration curves being performed using a chromatograph mass spectrometry device subjected to a first tuning by a standard sample and the measurement of the sample being detected being performed using a chromatograph mass spectrometry device subjected to a second tuning by a standard sample, and,
in the step of calculating the correction coefficient, the correction coefficient being calculated based on a signal intensity ratio of the substance being detected calculated from a first mass spectrum of the first tuning and an internal standard substance and a signal intensity ratio of the substance being detected calculated from a second mass spectrum of the second tuning and an internal standard substance and

that using this method, it is possible to correct fluctuations in detection sensitivity in analysis equipment and obtain high precision quantitatively determined values.

PTL 3 relates to a method of preparation of calibration curves in mass spectrometry. It describes a mass spectrometry device in which a known ratio of natural isotopes is utilized to simplify the preparation of the calibration curves, that, for example, carbon monoxide (CO) comes in four types of isotope substances of mass numbers 28, 29, 30, and 31 comprised of combinations of carbon (C) and oxygen (O), that the abundance ratio (concentration) of the four types of CO isotope substances is calculated based on the abundance ratio of isotopes of C and O, that the isotopes of CO are utilized as standard gases of four types of concentration, and that, due to this method, it is possible to prepare calibration curves without preparing large numbers of standard samples differing in concentration.

In this regard, the range in which quantitative analysis is utilized is broad. As explained above, it is utilized in the field of industry, the field of medical and life sciences, chemical analysis, etc. Among these, in production of ferrous materials and other metal materials, quantitative determination of the constituents in the metal is essential. In some ferrous material products, description of the constituents in the ferrous metals is sought in the mill sheets.

For the quantitative determination of nitrogen in iron and steel, several techniques are prescribed in JIS G 1228: 2006 (NPL 1). Among these, the inert gas melting-thermal conductivity method is being widely used as it offers high accuracy and reproducibility and enables analysis in a short time period. According to this method, the iron or steel sample covered is placed in graphite crucibles and rapidly heated in an inert gas stream using an impulse furnace to thereby vaporize the nitrogen from the iron and steel. The gaseous $N_2$ (nitrogen) is then quantitatively measured. At that time, as the inert gas, He (helium) is used. By using this as a carrier to the detector, it is possible to utilize the large difference in thermal conductivities of He and $N_2$ whereby a thermal conductivity detector (TCD) can be used for measurement with a high sensitivity.

In the above method, He is extremely important. He is industrially produced as a byproduct of LNG production. The main production is limited to specific regions and countries. Further, the price of He has continuously soared. Its future forecast is also unclear.

For this reason, development of a quantitative analysis method for the constituents in metal not using He has been sought. Compared with He, Ar (argon) is envisioned as an inexpensive inert gas promising stable supply. However, in the method disclosed in the above-mentioned NPL 1 of using a thermoconductivity analyzer to detect the gas constituents generated by heating in an impulse furnace, it was difficult to detect and quantitatively determine $N_2$ since $N_2$ and Ar are close in thermal conductivity.

PTL 4 proposes to avoid the use of He and quantitatively determine the nitrogen in steel in an Ar atmosphere by luminescence analysis. More specifically, the method of analysis of nitrogen in a metal sample of PTL 4 has a melting step of causing a metal sample containing a nitrogen constituent to melt in an argon atmosphere by electrical heating and causing the nitrogen constituent to vaporize and an analysis step of analyzing the nitrogen gas generated by the melting step and the argon gas by a gas discharge luminescence method to quantitatively determine the nitrogen in the metal sample.

The method of PTL 4 has as its object the quantitative determination of nitrogen in a metal. For quantitative determination of the other constituents, separate analysis was required. Further, for quantitative determination of nitrogen, oxidation, removal, and other operations on the other constituents generated along with melting of the sample (for example, CO, $CO_2$, $H_2$ etc.) were necessary, but the oxidation catalyst and de-$CO_2$ and dehydration materials used had to be replaced along with deterioration etc. On top of this, these operations were complicated. Further, in the calibration used as the grounds for quantitative determination of nitrogen, analysis using a standard sample was essential. The standard sample had to be managed.

In the above way, up to now, improvement of the mass spectrometry method has been studied in various ways. However, PTL 1 stops at eliminating the analysis step in specific applications. PTL 2 contributes to improvement of the precision of analysis, but the process is complicated and a longer time period becomes required for analysis. PTL 3 proposes to prepare a simple calibration curve by utilizing an isotope for detection, but preparation of the calibration curve requires separate measurement in the same way as the past. Further, none of these teaches or suggests not repeating preparation of calibration curves.

[CITATIONS LIST]

[PATENT LITERATURE]

[PTL 1] Japanese Unexamined Patent Publication No. 2017-215275
[PTL 2] Japanese Unexamined Patent Publication No. 2014-235088
[PTL 3] Japanese Unexamined Patent Publication No. 2000-065797
[PTL 4] Japanese Patent No. 5846344

[NONPATENT LITERATURE]

[NPL 1] JIS G 1228: 2006

SUMMARY

[TECHNICAL PROBLEM]

Mass spectrometers are generally high in sensitivity and are being broadly used. However, they easily fluctuate in sensitivity depending on the degree of vacuum of the vacuum tank etc., so calibration curves have had to be frequently prepared for sufficient accuracy and reproducibility.

The present invention was made considering the above and has as its object the provision of a method of quantitative analysis speeding up calibration and resistant to the effects of the condition of the device (fluctuations in sensitivity) in quantitative analysis using a mass spectrometer.

Further, in one aspect of the present invention, the measured object is not limited to nitrogen and He is not used, so a method and device enabling quantitative analysis of a measured object in a sample can be provided.

[SOLUTION TO PROBLEM]

The inventors came up with the idea of using the carrier gas itself which flows by a predetermined flow rate to the vacuum tank as the basis for the quantitative values to obtain a method for analysis for process control able to be routinely used without problems with variation in sensitivity of the mass spectrometer.

The inventors discovered the following points in this idea. A carrier gas is usually higher in concentration than the gas of the measured object, so exceeds the range of concentration to be measured. However, by analyzing mass peaks of natural isotopes of the carrier gas and using the natural isotopes as the standards for calibration, calibration by internal standards becomes possible.

For example, if making the gas of the measured object $N_2$ and using Ar gas as the carrier gas, the $^{40}$Ar accounting for the major part in the Ar gas becomes the carrier gas and the isotopes $^{36}$Ar and $^{38}$Ar can be used for preparation of calibration curves. The concentrations (abundance ratios) of the $^{36}$Ar and $^{38}$Ar in the Ar gas are respectively fixed concentrations of 3365 ppm and 652 ppm. Therefore, by analyzing the $^{36}$Ar and $^{38}$Ar contained in the carrier gas right in the middle of continuous measurement of the gas of the measured object using Ar gas as a carrier gas, calibration curves using these can be continuously found. For this reason, even if the sensitivity fluctuates, it is possible to continuously analyze the mass of $N_2$ of the gas of the measured object with a high accuracy and reproducibility.

It should be noted that, the ionizing potentials of $N_2$ and Ar are substantially the same ($N_2$ : 15.56Ev and Ar: 15.76eV). so it is possible to find the $N_2$ concentration from the calibration curves of $^{36}$Ar and $^{38}$Ar without adjustment by a correction coefficient. (If the ionizing potentials differ between the carrier gas and the gas of the measured object, it is possible to find a correction coefficient in advance by experiments so as to find the concentration of gas of the measured object.)

Furthermore, the inventors thought of applying the above idea to samples including metal. One example is a quantitative analysis method for taking the place of the conventional inert gas melting-thermal conductivity method. In general, the quantitative analysis method comprises melting a sample in an Ar or other inert atmosphere without using He, extracting the gas of the measured object from the sample, conveying the extracted gas to the mass spectrometry device, and quantitatively analyzing the measured object (for example, nitrogen, oxygen, hydrogen, etc.) in the sample by the device.

The Ar or other inert gas for melting the sample explained above can also be used as a carrier gas flowing through the mass spectrometry device. Isotopes of the carrier gas (argon etc.) can be used as the standards for calibration. Due to this, an analysis method for process control resistant to the effect of fluctuations in sensitivity of the mass spectrometer and able to be routinely used can be provided.

The present invention is based on the above findings. The following aspects are provided.

[1] A quantitative analysis method of a measured object using a mass spectrometer, which quantitative analysis method comprising

a gas introduction step of mixing a gas of the measured object and a carrier gas different from the gas of the measured object and having two or more natural isotopes and introducing them to a mass spectrometer,

a mass spectrometry step of mass spectrometry of the gas of the measured object and the two or more natural isotopes in the carrier gas at the mass spectrometer, and

a gas concentration calculation step of calculating a concentration of the gas of the measured object using analyzed values of the two or more isotopes of the carrier gas obtained by the mass spectrometry step as calibration standards.

[2] The quantitative analysis method according to [1], wherein the carrier gas contains at least one of Ar, $N_2$, and $O_2$ or a mixture of the same.

[3] The quantitative analysis method according to [1] or [2], further comprising an extraction step of heating a sample containing the measured object to make it melt in an atmosphere of the carrier gas and extracting the gas of the measured object from the sample.

[4] The quantitative analysis method according to [3], wherein the sample contains metal in 80 mass% or more.

[5] The quantitative analysis method according to any one of [1] to [4], further comprising a gas quantity calculation step of multiplying a concentration of the gas of the measured object and a flow rate of the carrier gas flowing in the time period in which the concentration was detected so as to calculate the amount of the gas of the measured object.

[6] The quantitative analysis method according to [5], further comprising, at the gas quantity calculation step, subdividing the time period in which the concentration of the gas of the measured object was detected, calculating the quantity Ci (i=1, 2,... n, where "n" is the number of subdivided sections) for each subdivided section, and calculating the total sum of Ci as the amount of the gas of the measured object.

[7] The quantitative analysis method according to any one of [1] to [6], wherein the two or more natural isotopes in the carrier gas are $^{36}$Ar and $^{38}$Ar.

[8] The quantitative analysis method according to any one of [1] to [7], wherein the measured object is one or more of any of nitrogen, carbon, oxygen, and hydrogen.

[9] The quantitative analysis method according to any one of [1] to [8], wherein the mass spectrometer has a resolution enabling separation and detection of mass number 28.006 of nitrogen gas and mass number 27.995 of carbon monoxide gas.

[10] The quantitative analysis method according to any one of [1] to [9], further comprising mass spectrometry of the gas of the measured object with a known concentration and the carrier gas in advance by the mass spectrometer to find a correction coefficient used for calculation of the concentration of the gas of the measured object .

[11] A quantitative analysis device comprising

a gas introduction function part for mixing a gas of a measured object and a carrier gas different from the gas of the measured object and having two or more natural isotopes and introducing them into a mass spectrometer,

a mass spectrometry function part provided at the mass spectrometer for mass spectrometry of the gas of the measured object and the two or more natural isotopes of the carrier gas, and

a gas concentration calculation function part for calculating a concentration of the gas of the measured object using as calibration standards the analysis values of the two or more natural isotopes of the carrier gas obtained at the mass spectrometry function part.

[12] The quantitative analysis device according to [11], further comprising a vaporization function part heating a sample containing the measured object to make it melt in an atmosphere of the carrier gas and render the measured object a gas from the sample.

[13] The quantitative analysis device according to [11] or [12], further comprising a gas concentration calculation function part using a signal intensity of the measured object measured by the mass spectrometry function part and the signal intensities of the two or more natural isotopes in the carrier gas to calculate the concentration of the gas of the measured object.

[14] The quantitative analysis device according to any one of [11] to [13], wherein the mass spectrometry function part has a resolution enabling separation and detection of the mass number 28.006 of nitrogen gas and the mass number 27.995 of carbon monoxide gas.

[15] The quantitative analysis device according to any one of [11] to [14] or [12], further comprising a chromatography function part able to separate the gas of the measured object and other gases.

[ADVANTAGEOUS EFFECTS OF INVENTION]

According to one embodiment of the present invention, a method of mass spectrometry speeding calibration and resistant to effects from the condition of the device (fluctuation in sensitivity) in mass spectrometry using a mass spectrometer, in particular, continuous process control, is provided. Specifically, it is possible to eliminate the preparation of calibration curves using standard gases and possible to reduce the costs required for analysis, in particular, the loss of time. In addition, since there is resistance to the effect of fluctuation in sensitivity due to fluctuation in the degree of vacuum, results of analysis having a high accuracy and reproducibility can be obtained. Further, the method of mass spectrometry can also be applied to continuous process control. Only naturally, it is possible to obtain results of analysis having a high accuracy and reproducibility even in that case.

It should be noted that, the accuracy and reproducibility in analysis can be suitably selected in accordance with the desired extents, the limiting conditions of the analysis device used, etc. In the embodiments of the present invention, "high accuracy" may be within tens of percent of the quantitatively determined value of the concentration envisioned (theoretically calculated value) or the quantitatively determined value forming the standard, typically within $\pm 30\%$, within $\pm 25\%$, within $\pm 20\%$, within $\pm 19\%$, within $\pm 18\%$, within $\pm 17\%$, within $\pm 16\%$, within $\pm 15\%$, within $\pm 14\%$, within $\pm 10\%$, within $\pm 5\%$, within $\pm 3\%$, within $\pm 2\%$, or within $\pm 1\%$. Alternatively, if the concentration is low, for example, if it is several tens of ppm or so or less than the same, "high accuracy" may be within tens of ppm of the quantitatively determined value envisioned (theoretically calculated value) or the quantitatively determined value forming the standard, typically within $\pm 90$ ppm, within $\pm 80$ ppm, within $\pm 70$ ppm, within $\pm 60$ ppm, within $\pm 40$ ppm, within $\pm 30$ ppm, within $\pm 20$ ppm, or within $\pm 10$ ppm. Further, in the embodiments of the present invention, "high reproducibility" may indicate the same extent of repeatable tolerance as the inert gas melting-thermal conductivity method prescribed in NPL 1, in particular Appendix 5. Note that, "repeatable tolerance" is the difference in the quantitative value when a single analysis person runs a test by the same device, under the same conditions, and by the same calibration curve within the shortest time. Typically, the repeatable tolerance is about 2 ppm to 550 ppm with respect to the standard concentration of 8 ppm to 5000 ppm of the standard substance (sample) (see NPL 1, in particular Appendix 5). Alternatively, "high reproducibility" may be a relative standard deviation of within tens of percent, typically may be within $\pm 20\%$, within $\pm 15\%$, within $\pm 12\%$, within $\pm 10\%$, within $\pm 8\%$, within $\pm 6\%$, within $\pm 5\%$, within $\pm 4\%$, within $\pm 3\%$, within $\pm 2\%$, or within $\pm 1\%$.

Further, according to one embodiment of the present invention, a method and device enabling quantitative analysis of a measured object in a sample, possibly one containing a metal etc., which does not use He and does not limit the target constituent to be determined to nitrogen are provided.

That is, the conventional inert gas melting-thermal conductivity method uses expensive He, but in one embodiment of the present invention, it is possible to use Ar or another inert gas more inexpensive and stabler in supply compared with He. This is because the present embodiment uses the mass spectrometry method , a general type of inert gas can be utilized for the carrier gas, and, further, such an inert carrier gas basically has no effect on fluctuations of sensitivity of the mass spectrometry.

Further, the carrier gas flowing through the mass spectrometer can also be utilized as the inert gas for melting the sample.

Furthermore, in one embodiment of the present invention, it is possible to use the high resolution mass spectrometry method. It becomes possible to eliminate pretreatment of the gas vaporized from the sample and to independently analyze several measured objects. Due to this, the various members, chemicals, etc. for pretreatment do not have to be prepared and replaced, the cost is lower, the structure is simpler, and a drop in measurement precision due to deterioration of these members etc. can be avoided.

Further, in one embodiment of the present invention, the carrier gas is Ar. The ionization rate of Ar is extremely stable. Therefore, by referring to measured values of the carrier argon gas isotopes and using them as standard values, it is possible to determine the concentration of the target constituent to be quantitatively determined in the sample gas. Due to this, calibration by standard samples can be made unnecessary. In particular, if the measured object also has a stable ionization rate, it is possible and preferable to suitably perform mass spectrometry. In other words, $N_2$ etc. having a stable ionization rate is a preferred measured object. Further, CO and $H_2O$, classified as inorganic gases, are also stable in ionization rates, so it is possible to make corrections for the differences in ionization energy to similarly perform quantitative analysis.

In addition, in one embodiment of the present invention, in quantitative analysis, the time period for measurement of the measured object is subdivided, the amount $C_i$ of the measured object is calculated for each subdivided measurement section, and the total sum of $C_i$ is calculated as the total amount of the measured object. Due to this, quantitative analysis with a further higher reproducibility (in other words, smaller variation in the measurement results) is possible.


BRIEF DESCRIPTION OF DRAWINGS


FIG. 1 is a view schematically expressing quantitative determination using conventional calibration curves.
FIG. 2 is a view showing the results of mass spectrometry of the gas of a measured object performed according to an embodiment of the present invention.

FIG. 3 is a view schematically expressing quantitative determination over time according to an embodiment of the present invention.

FIG. 4 shows a chart of the results of comparison of the prior art and invention examples.

FIG. 5 is a view schematically showing an outline of an analysis device.

FIG. 6 is a view showing an example of a high resolution mass spectrometry spectrum at which peaks of CO and $N_2$ are separated.

FIG. 7 is a view showing one example of measured values of $N_2$, $^{36}Ar$, and $^{38}Ar$ according to a high resolution mass spectrometry device.

FIG. 8 is a view showing the results of analysis of nitrogen according to an invention example.

FIG. 9 is a view showing one example of a mass spectrum.

FIG. 10 is a view showing one example of a mass spectrometry spectrum in the case using chromatography.

FIG. 11 is a view showing the schematic configuration of a quantitative analysis device of one embodiment of the present invention.

DESCRIPTION OF EMBODIMENTS

Below, a mass spectrometry method according to one embodiment of the present invention will be specifically explained.

The "mass spectrometry method according to one embodiment of the present invention" is a method of quantitative analysis of a measured object using a mass spectrometer.

A mass spectrometer is an device focusing on a mass of a gas of a measured object, identifying the gas of the measured object by the difference in mass, and performing quantitative determination from the detected intensity. In general, a mass spectrometer is comprised of a part for introduction of a measured object, an ionization part (ion source), a mass separation part (analyzer), a detection part (detector), a vacuum exhaust part (vacuum pump), a device control and data processing part (data system), etc. At the time of measurement, the introduced measured object is ionized at the ion source and rendered ions present in the gas phase. The ions differ in mobility depending on the mass to charge ratio (m/z), so various principles are used to separate them at an analyzer and detect them using a detector. The analyzer and detector are maintained at a degree of vacuum so that the ions will not strike other particles and separation and detection will not be impaired.

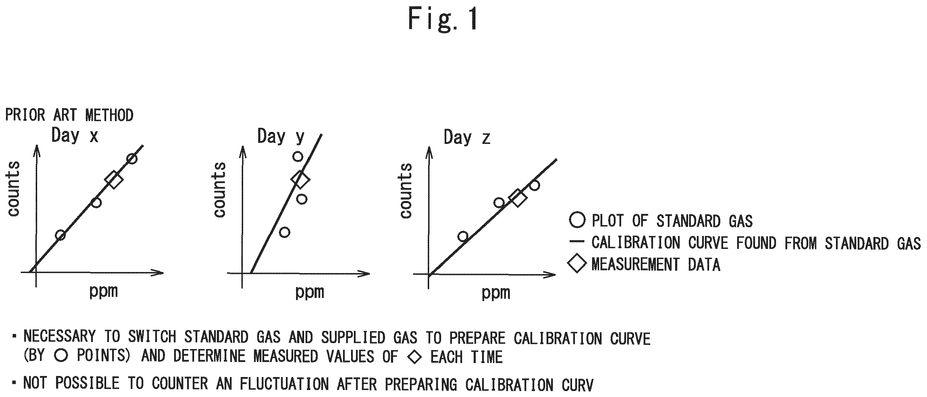

In the mass spectrometry method, in general, a calibration curve is used. Specifically, a standard sample of a known concentration is used to prepare a calibration curve in advance to quantitatively determine a measured object in an unknown sample. FIG. 1 schematically expresses quantitative determination using a conventional calibration curve. Despite the same standard sample being used on three measurement days (Days X, Y, Z), the calibration curves differ. This considered to be because the degree of vacuum etc. of the device fluctuates depending on the measurement date and has an effect on the sensitivity of the device. For this reason, for each measurement, it is necessary to prepare a calibration curve by a standard sample each time (by point ∘) to determine the measured value of an unknown sample (◊). Further, after preparing a calibration curve, if there is a fluctuation in the degree of vacuum in the mass spectrometer and a fluctuation in the sensitivity of the device accompanying that, these fluctuations cannot be countered and the accuracy and reproducibility of the quantitative determination sometimes are insufficient.

The mass spectrometry method according to one embodiment of the present invention comprises a method of quantitative analysis of a measured object using a mass spectrometer, comprising

a gas introduction step of mixing a gas of the measured object and a carrier gas different from the gas of the measured object and having two or more natural isotopes and introducing them to a mass spectrometer,

a mass spectrometry step of mass spectrometry of the gas of the measured object and the two or more natural isotopes in the carrier gas at the mass spectrometer, and

a gas concentration calculation step of calculating a concentration of the gas of the measured object using analyzed values of the two or more isotopes of the carrier gas obtained by the mass spectrometry step as calibration standards.

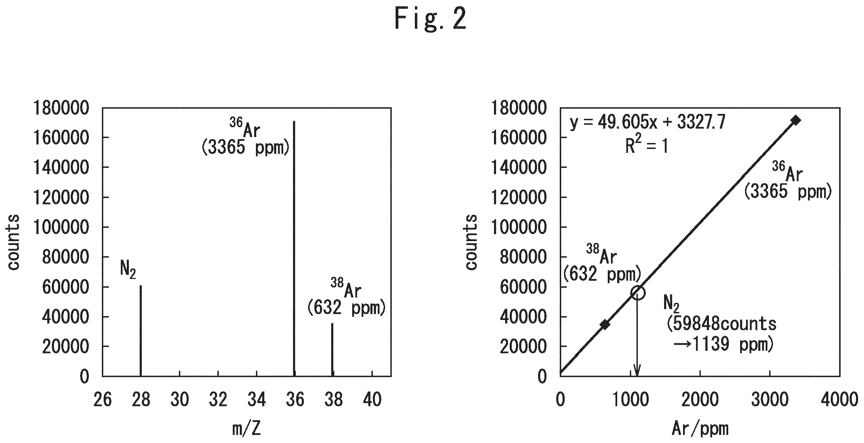

FIG. 2 shows an example of the results of mass spectrometry of the gas of a measured object performed in accordance with an embodiment of the present invention. More specifically, this shows the results of mass spectrometry in the case of mixing $N_2$ as the gas of the measured object and Ar as the carrier gas different from the gas of the measured object and having two or more natural isotopes, introducing them into a mass spectrometer, and quantitatively determining the $N_2$ of the gas of the measured object and the Ar of the two or more natural isotopes in the carrier gas. The Ar of the carrier gas is mostly $^{40}Ar$ and includes the isotopes $^{36}Ar$ and $^{38}Ar$. These isotopes can be used for preparation of calibration curves. The respective concentrations of the isotopes $^{36}Ar$ and $^{38}Ar$ are fixed concentrations of 3365 ppm and 652 ppm in accordance with the abundance ratio of isotopes. The calibration curves are prepared linking these known concentrations with the signal intensities (count) of the mass spectrum obtained by the mass spectrometer. The concentration of the gas of the measured object is calculated from the intensity (count) of the mass spectrum of the $N_2$ of the gas of the measured object using such a calibration curve. As a result, the concentration of $N_2$ is quantitatively determined.

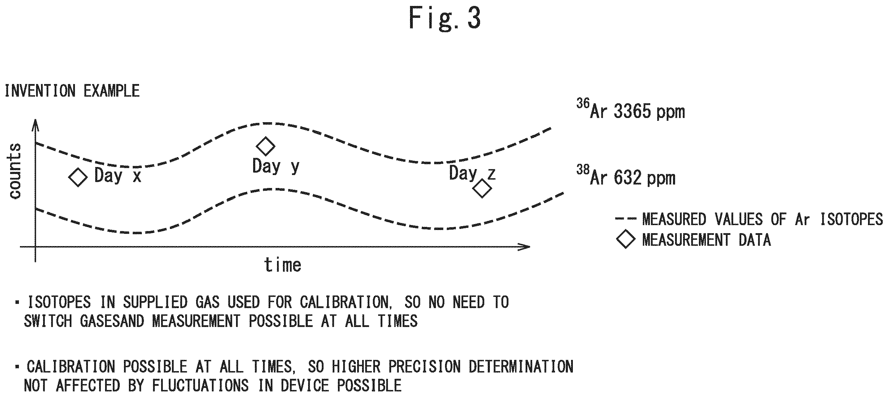

Further, the carrier gas is constantly circulating during measurement. The calibration curves can be continuously found. FIG. 3 schematically expresses quantitative determination along with time according to the embodiment of the present invention. Generally, quantitative determination on different days while using the same sample is expressed schematically. Along with the elapse of time, sometimes the sensitivity of detection of the mass spectrometer fluctuates. Along with fluctuation of the sensitivity of detection of the mass spectrometer, the signal intensity (count) of the mass spectrum of a natural isotope in the carrier gas also fluctuates. However, in the embodiment of the present invention, the natural isotopes in the carrier gas constantly circulate and the calibration curves can be changed in real time. Quantitative determination is possible using the latest calibration curves at all times. Therefore, quantitative determination resistant to the effects of fluctuation of sensitivity of the mass spectrometer and high in reproducibility is possible. Further, according to an embodiment of the present invention, it is also possible to eliminate the preparation of a calibration curve using a standard gas performed in the past for each mass analysis.

In one embodiment of the present invention, if the carrier gas differs from the gas of the measured object and has two or more natural isotopes, the two or more natural isotopes can be used as standard gases for mass spectrometry. However, since used as a carrier gas, it is preferably readily obtainable and low in cost. From this viewpoint, the carrier gas may be at least one of Ar, $N_2$, and $O_2$ or may be a mixture of the same. The present embodiment does not require use of helium as the carrier gas. It has great merit on this point as well. Typically, the two or more natural isotopes may include [36] Ar and [38] Ar. If more than two natural isotopes are present, it is also possible to use three or more natural isotopes as standard gases for mass spectrometry. Due to this, it is possible to raise the accuracy and reproducibility of the mass spectrometry more. Here, a calibration curve may be a straight line passing through the origin at concentration 0% or other one found by suitable fitting. Such a step may be referred to as a "mass spectrometry step."

One embodiment of the present invention may include an extraction step of heating a sample containing the measured object in an atmosphere of a carrier gas to make it melt and extract a gas of the measured object from the sample. It should be noted that, the carrier gas of the present embodiment may also be an inert gas. This is to avoid oxidation or other reaction of the measured object contained in the sample when heating the sample to melt. From this viewpoint, as the inert carrier gas, nitrogen, argon, etc. may be used. Due to this embodiment, it is possible to perform the process from extraction of the measured object from the sample to analysis of the measured object at the mass spectrometer continuously. This can also be applied to continuous process control and is preferable.

By heating a sample containing a measured object to melt in an atmosphere of a carrier gas, the measured object which was contained in the sample vaporizes and is extracted. For example, the nitrogen, carbon, oxygen, hydrogen, etc. in a sample are respectively vaporized to nitrogen gas, carbon monoxide gas, carbon dioxide gas, oxygen gas, hydrogen gas, etc. In general, if using a graphite crucible for the crucible at the time of heating, the oxygen gas reacts with the carbon to produce carbon monoxide. Further, in this Description, generation of a gas accompanying a reaction is also defined broadly as "vaporization."

As the means for heating a sample to melt, an impulse furnace may be used. Typically, an impulse furnace is provided inside it with a crucible comprised of graphite etc. and electrodes for holding the crucible and heating it. By charging the sample into this crucible and applying a current to the electrodes, the sample in the crucible is quickly heated and melts and the measured object contained in the sample can also be vaporized. A supply line for carrier gas may also be provided for rendering the inside of the impulse furnace, in particular the atmosphere around the sample, an atmosphere of the carrier gas.

The gas extracted from inside the sample is introduced to the mass spectrometer along with the stream of the carrier gas. Such a step may also be called the "gas introduction step." The stream of carrier gas introduced to the mass spectrometer may be one utilizing the carrier gas in the extraction step or one supplied separately from that or one mixing the two. Between the extraction step function part performing the extraction step or the vaporization function part (for example, impulse furnace) and mass spectrometer, a pressure regulator may be provided. The flow rates of the extracted gas of the measured object and stream of carrier gas may be controlled by the pressure difference. Further, a flow meter may also be provided for monitoring the flow rate, and regulation by the pressure regulator may be controlled in accordance with the measured flow rates. Further, at the upstream side from the pressure regulator or mass spectrometer, a dust filter may be provided. Due to the dust filter, the dust produced in the extraction step or vaporization step (for example, impulse furnace) can be removed and equipment at the downstream side (pressure regulator, mass spectrometry device, etc.) can be protected. As the dust filter, for example, a filter with good ventilation made of silica fibers or polytetrafluoroethylene can be used.

The sample contains a measured object. The measured object is not particularly limited so long as one which can be extracted by heating and melting. The sample may also contain a metal. Typically, the sample may contain 80 mass% or more of metal. It should be noted that, "metal" indicates an element positioned downward to the left of the slanted line connecting boron (B) and astatine (At) on the periodic table. That is, the "metal" may include, by element symbols, B, Na, Mg, Al, Si, K, Ca, Sc, Ti, V, Cr, Mn, Fe, Co, Ni, Cu, Zn, Ga, Ge, As, Rb, Sr, Y, Zr, Nb, Mo, Tc, Ru, Rh, Pd, Ag, Cd, In, Sn, Sb, Te, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, Po, At, Fr, Ra, Ac, Th, Pa, U, Np, Pu, Am, Cm, Bk, Cf, Es, Fm, Md, No, Lr, Rf, Db, Sg, Bh, and Hs. In a sample, in actuality, in addition to the matrix metal and other metal constituents as alloy constituents, nitrogen, carbon, oxygen, hydrogen, and various other (nonmetallic) constituents are sometimes included. These may also be the measured objects. Typical examples of the sample may include reduced iron, metal samples, ferrous metal samples, alloy samples, etc.

The quantitative analysis of one embodiment of the present invention can calculate the concentration of the gas of the measured object. Such a step may also be called the "gas concentration calculation step." Based on this, it is also possible to calculate the amount of the gas of the measured object conveyed by the carrier gas. That is, by multiplying the concentration of the gas of the measured object and the flow rate of the carrier gas flowing in the time period in which that concentration was detected, it is possible to calculate the amount of the gas of the measured object. This step may also be referred to as the "gas quantity calculation step." It is also possible to calculate the amount of the gas of the measured object in the sample. The amount of gas may be converted to volume or may be converted to mass etc.

In the gas quantity calculation step, the time period in which the concentration of the gas of the measured object is detected may be subdivided, the quantity $C_i$ ($i=1, 2,... n$, where "n" is the number of subdivided sections) of the gas of the measured object may be calculated for each subdivided section, and the total sum of $C_i$ may be calculated as the amount of the gas of the measured object.

The subdivided sections may be determined in accordance with the measurement frequency of the mass spectrometer (measurement interval). Due to this, it is possible to calculate the volume of the gas of the measured object from the concentration and measurement frequency (measurement interval). Typically, the concentration of the gas of the measured object measured at the mass spectrometer at a certain measurement timing is multiplied with the flow rate of carrier gas flowing in the section (in other words, measurement interval) from that measurement timing (measurement point) to the next measurement timing (measurement point) so as to calculate the volume of the gas of the measured object at the measurement interval (interval from a certain measurement point to the next measurement point). These are cumulatively added. More specifically, in the mass spectrometer, measurement may be conducted (further, the signal intensity may be fetched) by a measurement frequency of 10 to 1200 times/min or so. At each measurement timing (measurement point), the signal intensity obtained at that measurement timing (measurement point) may be used to update the calibration curves of the natural isotopes, calculate the gas of the concentration of the measured object, and in turn find the amount $C_i$ of the gas of the measured object at each measurement interval (interval from a certain measurement point to a next measurement point). The total sum of the cumulatively added amounts $C_i$ of the gas of the measured object for each measurement timing (measurement point) corresponds to the amount of the gas of the measured object and the sample.

By this subdivision, the accuracy and reproducibility of quantitative determination can be further improved. For example, compared with the case of using a calibration curve of an isotope obtained a few seconds before when the gas of the measured object started being detected, the results of quantitative analysis obtained by subdivision can be further improved in accuracy and reproducibility. In other words, it is possible to obtain results of quantitative analysis with small variation in the results of quantitative analysis.

The flow rate of the carrier gas and the measurement frequency (measurement interval) of the mass spectrometer are not particularly limited. In general, the greater the number of times of measurement per certain time period (measurement points) of an isotope and target constituent of quantitative determination, in other words, the higher the measurement frequency and the shorter the measurement interval, the smaller the variation in the results of quantitative analysis. For this reason, the number of times of measurement (measurement points) may be increased. In other words, the measurement frequency may be raised (the measurement interval may be shortened). It is also possible to select it in accordance with the accuracy etc. sought for quantitative determination in the range of a typical measurement frequency of 10 to 1200 times/min. Further, the flow rate of the carrier gas may be suitably selected in accordance with the conditions of use of the mass spectrometer. Typically, it may be selected in the range of 10 to 10000 mL/min.

In one embodiment, the measured object may be one or more of nitrogen, oxygen, and hydrogen. Mass spectrometry has the merit of being able to identify and quantitatively determine with a high accuracy a large number of gas constituents by one analysis (measurement) compared with other techniques (for example, the inert gas melting-thermal conductivity method etc.) For this reason, the measured object is not limited to nitrogen and may also be one or more of any of nitrogen, carbon, oxygen, and hydrogen. Typically, constituents other than nitrogen, for example, carbon monoxide etc. can be determined simultaneously with nitrogen.

In one embodiment, the mass spectrometer may have a resolution enabling separation and detection of the mass number 28.006 of nitrogen gas and the mass number 27.995 of carbon monoxide gas. The theoretically required resolution of CO and $N_2$ is considered to be $R=27.995/(28.006-27.995)=2545$, but in practice, a higher one is desirable. On the other hand, from the relationship with sensitivity, sufficient accuracy is obtained by a resolution of $R=25000$ or less, so typically the resolution R may be 20000 or so. Due to this, at the mass spectrometer, it is possible to separate the nitrogen gas and carbon monoxide gas and obtain results of quantitative determination having sufficient accuracy. In other words, at the steps before analysis by the mass spectrometer, separation of nitrogen gas and carbon monoxide gas is unnecessary and simplification of the analysis procedure and analysis at lower cost become possible, so this is preferable. Further, if separating the nitrogen gas and carbon monoxide gas at the steps before quantitative analysis by the mass spectrometer or if the content of carbon in the sample is low and there is no effect by carbon monoxide, i.e., if there is no effect on measurement of the signal intensity of the mass number 28.006 of the nitrogen gas, a mass spectrometer not having the above resolution may also be used.

In mass spectrometry, the following correction may be performed. In mass spectrometry, it is essential to ionize the introduced sample by an ion source and render it ions present in a gas phase. Here, the ionization potential differs depending on the substance. For this reason, if the ionization potentials of the gas of the measured object and the standard gas (two or more natural isotopes) are equal, it is possible to efficiently ionize these by a single ion source. In other words, there is no need to correct the quantitatively determined value of the gas of the measured object found from a calibration curve of the standard gas. However, the ionization potential differs between gas of the measured object and the standard gas (two or more natural isotopes). If there is an effect on the accuracy of the quantitatively determined value, a correction coefficient for correcting the quantitatively determined value may be found by tests. By using this correction coefficient, it is possible to raise the accuracy of quantitative determination of the gas of the measured object. Typically, it is possible to quantitatively analyze a gas of a measured object with a known concentration and a carrier gas in advance and use the ratio of the known concentration to the measured quantitatively determined value as the correction coefficient. Further, if the carrier gas is a mixed gas, it is preferable to use a gas with an equal ionization potential and not requiring a correction coefficient as the standard gas. However, depending on the concentration of the gas of the measured object, a gas requiring a correction coefficient or several types of gas may be used as the standard gas. It should be noted that, nitrogen has an ionization potential substantially the same as argon ($N_2$ : 15.56eV and Ar: 15.76eV). It is possible to find the $N_2$ concentration from the calibration curves of $^{36}$Ar and $^{38}$Ar without adjustment by a correction coefficient. That is, nitrogen and argon are one example of a preferable combination of a measured object and a carrier gas.

According to one embodiment of the present invention, a quantitative analysis device (110) is provided.

The quantitative analysis device (110) has

an introduction function part (111) for mixing a gas of a measured object and a carrier gas different from the gas of the measured object and having two or more natural isotopes and introducing them into a mass spectrometer (112), a mass spectrometry function part (112-1) provided at the mass spectrometer (112) for mass spectrometry of the gas of the measured object and the two or more natural isotopes of the carrier gas, and a gas concentration calculation function part (113) for calculating a concentration of the gas of the measured object using as calibration standards the analysis values of the two or more natural isotopes of the carrier gas obtained at the mass spectrometry function part (112-1).

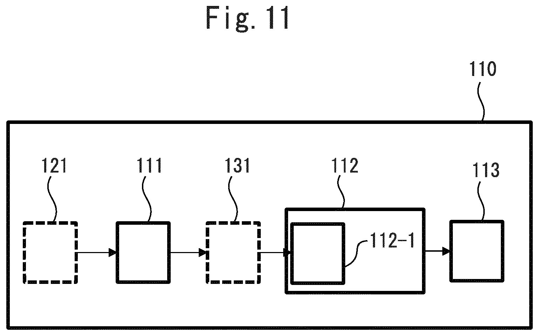

FIG. 11 shows the schematic configuration of the quantitative analysis device (110) of the present embodiment. The broken line block at FIG. 11 shows optionally added component elements.

According to this device, it is possible to suitably perform the quantitative analysis method of one embodiment of the present invention.

In one embodiment, a vaporization function part (121) which heats a sample including a measured object in an atmosphere of a carrier gas to make it melt and render the measured object a gas from the sample may be provided at the quantitative analysis device (110). According to the present embodiment, the entire process from the vaporization or extraction of the measured object from the sample to analysis of the measured object at the mass spectrometer (112) can be performed continuously. This can be applied to a continuous process as well and therefore is preferable.

In one embodiment, a gas concentration calculation function part (113) using a signal intensity of a measured object measured by the mass spectrometry function part (112-1) and the signal intensities of two or more natural isotopes in the carrier gas to calculate the concentration of the gas of the measured object may be provided at the quantitative analysis device (110). The gas concentration calculation function part (113) may also be comprised of a computer performing processing operations.

The present embodiment shows a typical processing routine inside the mass spectrometry device (110). At the mass spectrometry function part (112-1), the signal intensity of the measured object and the signal intensity of two or more natural isotopes in the carrier gas are measured. At the gas concentration calculation function part (113), the concentration of gas of the measured object is calculated using the signal intensities measured at the mass spectrometry function part (112-1).

In one embodiment, the mass spectrometry function part (112-1) may have a resolution enabling separation and detection of the mass number 28.006 of nitrogen gas and the mass number 27.995 of carbon monoxide gas. The theoretically required resolution of CO and $N_2$ is considered to be R=27.995/(28.006-27.995)=2545, but in practice, a higher one is desirable. On the other hand, from the relationship with sensitivity, sufficient accuracy is obtained by a resolution of R=25000 or less, so typically the resolution R may be 20000 or so. Due to this, at the mass spectrometry function part (112-1), it is possible to separate the nitrogen gas and carbon monoxide gas and obtain results of quantitative determination having sufficient accuracy. In other words, at the steps before analysis by the mass spectrometry function part (112-1), separation of nitrogen gas and carbon monoxide gas is unnecessary and simplification of the analysis procedure and analysis at lower cost become possible, so this is preferable. Further, if separating the nitrogen gas and carbon monoxide gas at the steps before quantitative analysis by the mass spectrometry function part (112-1) or if the content of carbon in the sample is low and there is no effect by carbon monoxide, i.e., if there is no effect on measurement of the signal intensity of the mass number 28.006 of the nitrogen gas, a mass spectrometry function part (112-1) not having the above resolution may also be used.

In another embodiment, the mass spectrometry device (110) may also have a chromatography function part (131) able to separate the gas of the measured object and gases other than the same. It is also possible to segment the measured object by running it through chromatography before introducing the measured object into the mass spectrometer (112). Due to this, it is possible to further improve the accuracy of the quantitative analysis. The configuration of the chromatography function part (131) may be suitably selected or adjusted in accordance with the object desired to separate. In a typical example, it is also possible to use chromatography able to separate carbon monoxide and nitrogen over time. Due to this, it is possible to separate the nitrogen gas and carbon monoxide gas close in mass numbers, then introduce them into the mass spectrometer (112). As a result, it is possible to distinguish two gases and obtain results of determination with high accuracy for these. The results of quantitative analysis according to the present embodiment substantially match with the results of quantitative analysis according to wet chemical analysis. On the other hand, some difference is observed in the results of quantitative analysis by the inert gas melting-thermal conductivity method with the results of quantitative analysis by wet chemical analysis. On this point, the quantitative analysis according to the present embodiment can be given an accuracy higher than the inert gas melting-thermal conductivity method.

EXAMPLES

Below, the present invention will be explained in more detail using examples. However, the present invention should not be interpreted limited to the examples.

[Example I]

(Example I-1) Carrier Gas: Ar, Gas of Measured Object: $N_2$

A high resolution mass spectrometer (HV-infiTOF) made by MSI-TOKYO was used for quantitative analysis of $N_2$ gas using Ar gas as the carrier gas and $N_2$ gas as the gas of the measured object. Further, the following three techniques were compared.

(1) Preparation of calibration curve each time (prior art)
(2) Continuous use of calibration curve prepared at time of start of the test (prior art)
(3) Determination based on ratio of natural isotopes of carrier gas (Ar gas) (invention example)

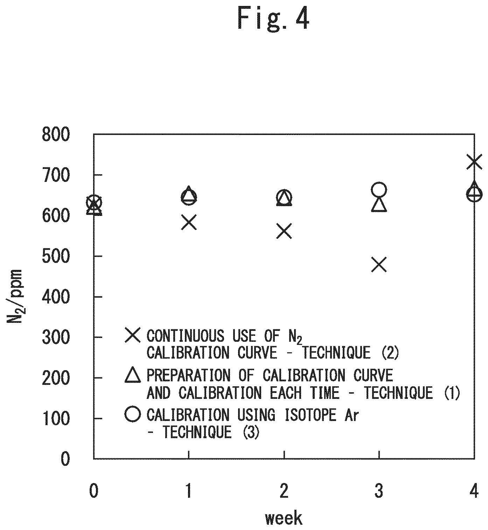

$N_2$ gas was mixed in a 600 to 700 ppm range with respect to the carrier gas Ar gas 1000 ml/min. Every week, the $N_2$ concentration was analyzed. FIG. 4 shows the results of comparison of the prior art (techniques (1) and (2)) and the invention examples (technique (3)).

The calibration curves used in the techniques (1) and (2) were prepared from the $N_2$ concentrations and the analyzed values of the mass spectrometer obtained by measuring mixed gas of Ar gas and $N_2$ gas with different $N_2$ concentrations ($N_2$ concentration: 100 ppm, 800 ppm, 1500 ppm) by a high resolution mass spectrometer.

In this test, about 2 hours or so were required for the work of preparing this calibration curve. If performing the work of preparing a calibration curve for each measurement, the maximum/minimum error (difference between maximum value and minimum value) was about 20 ppm or so (technique (1)). If continuing to use the first prepared calibration curve, a large difference arises in the measured values and sometimes the maximum/minimum error became 100 ppm or more (technique (2)).

In the invention example, the $^{36}$Ar and $^{38}$Ar measured simultaneously at the time of measurement of the $N_2$ gas were assumed to be the ratio of natural isotopes of the concentration 3365 ppm and concentration 632 ppm, and formula 1 was used to calculate the $N_2$ concentration (technique (3)).

N2 concentration (ppm)=N2 peak intensity$\times$(3365 ppm-632 ppm)/($^{36}$Ar peak intensity-$^{38}$Ar peak intensity)... (formula 1)

(formula 1)

In the present invention, the maximum/minimum error is shown as 20 ppm or so or a precision of determination equal to the technique (1). On the other hand, in the invention examples, no work for making the standard gas circulate for calibration arose, so no loss in time occurred.

(Example I-2) Carrier Gas: Ar, Gas of Measured Object: $O_2$, CO, $CO_2$

Analysis in the case of making the carrier gas Ar gas and making the gas of the measured object $O_2$, CO, $CO_2$ gases was studied. Introduced gas in which $O_2$, CO, $CO_2$ were contained (air ($O_2$ concentration 20.9%) in which CO was mixed to concentration 1000 ppm and $CO_2$ was mixed to concentration 2000 ppm) was mixed with carrier gas 1000 ml/min in seven test levels of 250, 200, 150, 100, 50, 30, 15 ml/min for analysis. For the natural isotopes of the carrier gas used for calibration, the peaks of $^{36}$Ar, $^{38}$Ar were used. From the ratio of natural isotopes, the $^{36}$Ar, $^{38}$Ar were deemed a concentration of 3365 ppm and a concentration of 632 ppm, and formula 2 was used to calculate the concentrations of the various gases. The results are shown in Table 1. Even with different types of gases mixed in the gas of the measured object, it could be confirmed that analyzed values are shown by a high accuracy.

Concentration of gas of measured object (ppm) = Peak intensity of gas of measured object $\times$(3365 ppm-632 ppm)/($^{36}$Ar peak intensity-$^{38}$Ar peak intensity)... (formula 2)          (formula 2)

[Table 1]

| Table 1. Calculated Concentrations and Results of Analysis Based on Flow Rates of $O_2$, CO, and $CO_2$ gas | | | | | | | |
|---|---|---|---|---|---|---|---|
| Flow rate of carrier gas | Flow rate of introduced gas | $O_2$ concentration (ppm) | | CO concentration (ppm) | | $CO_2$ concentration (ppm) | |
| (ml/min) | (ml/min) | Calculated concentration | Results of analysis | Calculated concentration | Results of analysis | Calculated concentration | Results of analysis |
| 1000 | 250 | 41800 | 41659 | 200 | 192 | 400 | 422 |
| 1000 | 200 | 34833 | 36093 | 167 | 153 | 333 | 336 |
| 1000 | 150 | 27261 | 28768 | 130 | 131 | 261 | 269 |
| 1000 | 100 | 19000 | 17934 | 91 | 90 | 182 | 181 |
| 1000 | 50 | 9952 | 9857 | 48 | 47 | 95 | 85 |
| 1000 | 30 | 6087 | 6394 | 29 | 29 | 58 | 62 |
| 1000 | 15 | 3089 | 3579 | 15 | 13 | 30 | 29 |

(Example I-3) Carrier Gas: $O_2$, Gas of Measured Object: Ar

Analysis in the case of making the carrier gas $O_2$ and making the gas of the measured object Ar gas was studied. Introduced gas in which Ar was contained (Ar: 1.0%, $N_2$ : 99.0%) was mixed with carrier gas 1000 ml/min in seven test levels of 10.0, 8.0, 6.0, 4.0, 2.0, 1.0, and 0.5 ml/min for analysis. For the natural isotopes in the carrier gas used for calibration, the peaks of $^{17}O^{16}O$, and $^{18}O^{16}O$ showing suitable peak intensities were used. From the ratio of the natural isotopes, it was assumed that $^{17}O^{16}O$, and $^{18}O^{16}O$ were present in concentrations of 380 ppm and 2046 ppm. Formula 3 was used to find the concentration of Ar. The results are shown in Table 2. It could be confirmed that analyzed values were shown for the mixed concentrations by an accuracy similar to Examples I-1 and 2.

Ar concentration (ppm) = Peak intensity of gas of measured object $\times$ (2046 ppm-380 ppm)/($^{18}O^{16}O$ peak intensity-$^{17}O^{16}O$ peak intensity)... (formula 3)          (formula 3)

[Table 2]

| Table 2. Calculated Concentrations and Results of Analysis Based on Flow Rate of Ar Gas | | | |
|---|---|---|---|
| Flow rate of carrier gas | Flow rate of introduced gas | Ar concentration (ppm) | |
| (ml/min) | (ml/min) | Calculated concentration | Results of analysis |
| 1000 | 10.0 | 99.0 | 101.0 |
| 1000 | 8.0 | 79.4 | 76.6 |
| 1000 | 6.0 | 59.6 | 61.0 |
| 1000 | 4.0 | 39.8 | 38.0 |
| 1000 | 2.0 | 20.0 | 20.9 |
| 1000 | 1.0 | 10.0 | 9.7 |
| 1000 | 0.5 | 5.0 | 5.4 |

(Example I-4) Carrier gas: $N_2$, Gas of Measured Object: Ar

Analysis in the case of making the carrier gas $N_2$ and making the gas of the measured object Ar gas was studied. Introduced gas in which Ar was contained (Ar: 1.0%, $N_2$ : 99.0%) was mixed with carrier gas 1000 ml/min in seven test levels of 10.0, 8.0, 6.0, 4.0, 2.0, 1.0, and 0.5 ml/min for analysis. For the natural isotopes in the carrier gas used for calibration, the peak of $^{15}N^{14}N$ showing a suitable peak intensity was used. Further, in this experiment, there were no natural isotopes having excellent peak intensities other than $^{15}N^{14}N$, so it was judged that a concentration of 0% was a peak intensity of 0 and only $^{15}N^{14}N$ was quantitatively determined. From the ratio of the natural isotopes, it was assumed that $^{15}N^{14}N$ was present in a concentration of 3629 ppm. Formula 4 was used to find the concentration of Ar. The results are shown in Table 3. It could be confirmed that analyzed values were shown for the mixed concentrations by an accuracy similar to Examples I-1, 2, and 3.

Ar concentration 0 (ppm)=Peak intensity of gas of measured $\times$ (3692 ppm/$^{15}N^{14}N$ peak intensity)...  (formula 4)
(formula 4)

[Table 3]

| Table 3. Calculated Concentrations and Results of Analysis Based on Flow Rate of Ar Gas | | | |
|---|---|---|---|
| Flow rate of carrier gas | Flow rate of introduced gas | Ar concentration (ppm) | |
| (ml/min) | (ml/min) | Calculated concentration | Results of analysis |
| 1000 | 10.0 | 99.0 | 105.5 |
| 1000 | 8.0 | 79.4 | 84.0 |
| 1000 | 6.0 | 59.6 | 56.1 |
| 1000 | 4.0 | 39.8 | 45.2 |
| 1000 | 2.0 | 20.0 | 19.4 |
| 1000 | 1.0 | 10.0 | 10.3 |
| 1000 | 0.5 | 5.0 | 3.7 |

(Example I-5) Carrier Gas: Ar, Gas of Measured Object: $N_2$, He

Analysis in the case of making the carrier gas Ar and making the gas of the measured object $N_2$ and He was studied. Introduced gas in which $N_2$ and He were contained (He: 3000 ppm, $N_2$ : 3000 ppm, $O_2$ : 99.4%) was mixed with a carrier gas 1000 ml/min in seven test levels of 10.0, 9.0, 7.0, 5.0, 3.0, 2.0, and 1.0 ml/min and analyzed. For the natural isotopes of the carrier gas used for calibration, the peaks of $^{36}Ar$, $^{38}Ar$ were used. From the ratio of the natural isotopes, it was assumed that the $^{36}Ar$, $^{38}Ar$ were present in a concentration 3365 ppm and concentration 632 ppm. Formula 5 was used to calculate the concentrations of $N_2$ and He. The results are shown in Table 4. $N_2$ could be analyzed for accurate concentration in the same way as Examples I-1 to 1-4, but the concentration of He did not match the concentration calculated based on the flow rate. This is believed to be due to the difference in ionization potential of He and Ar. The ionization potential of He is 24.587eV, and the ionization potential of Ar is 15.76eV. He requires large energy for ionization. Under the same electron impact conditions in mass spectrometry, it is believed that the measured value fell due to the presence of molecules which were not ionized.

Concentration of gas of measured object (ppm)=Peak intensity of gas of measured object$\times$(3365 ppm-632 ppm)/($^{3\,6}$Ar peak intensity-$^{3\,8}$Ar peak intensity)... (formula 5)　　　(formula 5)

[Table 4]

| Table 4. Calculated Concentrations and Results of Analysis Based on Flow Rates of $N_2$ and He Gas | | | | | | |
|---|---|---|---|---|---|---|
| Flow rate of carrier gas | Flow rate of introduced gas | N2 concentration (ppm) | | He concentration (ppm) | | |
| (ml/min) | (ml/min) | Calculated concentration | Results of analysis | Calculated concentration | Results of analysis | Results of analysis $\times$1.2 |
| 1000 | 10.0 | 29.7 | 30.0 | 29.7 | 24.2 | 29.0 |
| 1000 | 9.0 | 26.8 | 26.0 | 26.8 | 21 | 25.2 |
| 1000 | 7.0 | 20.9 | 20.5 | 20.9 | 16.7 | 20.0 |
| 1000 | 5.0 | 14.9 | 14.3 | 14.9 | 13.6 | 16.3 |
| 1000 | 3.0 | 9.0 | 9.7 | 9.0 | 8.9 | 10.7 |
| 1000 | 2.0 | 6.0 | 5.1 | 6.0 | 3.7 | 4.4 |
| 1000 | 1.0 | 3.0 | 2.7 | 3.0 | 2.1 | 2.5 |

Regarding the results, by multiplying the results of analysis of the He concentration by 1.2, it was confirmed that the calculated concentration based on the flow rate and the analysis values matched well.

From the above results, it was learned that if making a gas with a higher ionization potential than the carrier gas the gas of the measured object, the accuracy of the analysis value is raised by correction by multiplying the analyzed value with a constant. In the present test, the correction constant was 1.2, but it changes depending on the intensity of the electron impact, so is not limited to this value

(Example I-6) Carrier Gas: Ar, Sample Gas: $N_2$

Analysis in the case of making the carrier gas Ar and making the gas of the measured object $N_2$ was studied. $N_2$ gas was injected (mixed) in seven test levels of 2.0, 5.0, 10.0, 15.0, 20.0, 25.0, and 30.0$\mu$L with respect to the carrier gas 400 mL/min, and the amount of injection (volume) was analyzed from the concentration and time. The concentration of $N_2$ was measured by a frequency of 300 times/min. For the isotopes of the carrier gas used for calibration, the peaks of $^{3\,6}$Ar, $^{3\,8}$Ar were used. The respective concentrations were made 3365 ppm and 632 ppm from the ratio of natural isotopes. The result of measurement of concentration in the time period from when the $N_2$ gas is introduced to when the $N_2$ gas is discharged from the mass spectrometer by the carrier gas are added together using formula 6 to calculate the amount of injection. The results are shown in Table 5. Under conditions where the concentration fluctuates as well, results of analysis can be found by an accuracy similar to the other examples and the amount of injection can be analyzed.

Amount of $N_2$ injected ($\mu$L)=$\Sigma${$N_2$ peak intensity$\times$(3365 ppm-632 ppm)/($^{3\,6}$Ar peak intensity-$^{3\,8}$Ar peak intensity)$\times$(carrier gas flow rate (mL/min.))/frequency of measurement of concentration (times/min.)}$\times$1000...(formula 6)　　　(formula 6)

[Table 5]

| Table 5. Amount of Injection and Results of Analysis of $N_2$ Gas | |
| --- | --- |
| Amount of injection | Results of analysis |
| ($\mu$L) | ($\mu$L) |
| 30.0 | 30.0 |
| 25.0 | 26.0 |
| 20.0 | 20.5 |
| 15.0 | 14.3 |
| 10.0 | 9.7 |
| 5.0 | 5.1 |
| 2.0 | 2.7 |

[Example II]

The mass spectrometer was provided with the function of heating a sample containing the measured object to melt and vaporize (extract) the measured object from the sample and quantitatively analyzed the measured object.

(Hardware Configuration)

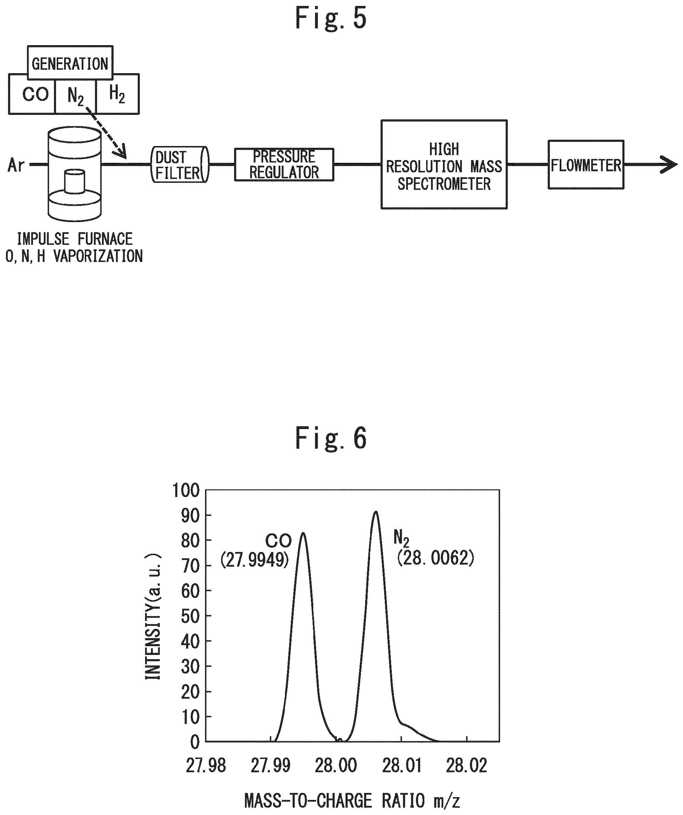

To enable the analytical tests of the examples and comparative examples, an oxygen, nitrogen, and hydrogen analysis device EMGA930 made by Horiba Ltd. was modified, the carrier gas was made argon gas, and a pressure regulator and flowmeter were added right after the dust filter. This device was directly connected with a multiturn type high resolution mass spectrometer infi-TOF-UHV made by MSI.TOKYO to prepare a test device. FIG. 5 shows a schematic view of the test device.

(Measurement Samples)

As the measurement samples, three levels of standard samples of ferrous metal made by Seishin Trading Co., Ltd., two levels of standard samples of ferrous metal of JSS, a standard sample of titanium made by Seishin Trading Co., Ltd., and six levels of reduced iron were prepared. Further, measurement at only the crucible was made a blank (sample). These samples were measured in the following way, in particular, were determined in amounts of nitrogen contained in the samples. Table 6 shows the amounts of nitrogen contained in the standard samples of ferrous metal (certified value).

[Table 6]

| Table 6. Measurement Samples | |
| --- | --- |
| Sample name | N (mass ppm) |
| JSS 366-9 | 6.5 |
| JSS 367-9 | 44 |
| SS-2-98 | 19.5 |
| SS-3-51 | 61.0 |
| SS-4-94 | 144.6 |

(Preliminary Analysis)

The above standard sample of a ferrous metal was weighed, charged into a graphite crucible, and heated to vaporize in the impulse furnace of the modified device. The gas generated was detected by a high resolution mass spectrometer. The mass spectrum of the gas measured was checked, whereupon the nitrogen-based gas generated from the sample was only $N_2$. It was confirmed that the oxygen generated from the sample was detected only as the CO generated by oxidation of the graphite crucible.

Therefore, in the following examples and comparative examples, if determining the amount of the nitrogen in a standard sample of ferrous metal through quantitative determination of the amount of $N_2$, the peaks of CO and $N_2$ have to be separated. High resolution mass spectrometry required for separating and measuring the respective precision masses of 28.0062 and 27.9949 was performed. FIG. 6 shows an example of a high resolution mass spectrometry spectrum with peaks of CO and $N_2$ separated. The present invention is not limited to a resolution giving this spectrum. The resolution R of the device when this spectrum was obtained was about 20000.

(Example II-1)

The following analysis was performed using a test device (FIG. 5).

Three levels of standard samples of ferrous metal made by Seishin Trading Co., Ltd., two levels of standard samples of ferrous metal of JSS, and a blank were respectively measured N=10 times.

Using (formula II-1), the amount of $N_2$ was determined based on the concentrations of Ar isotopes in the carrier gas. Explaining the points of (formula II-1), the concentrations of the isotopes $^{36}$Ar and $^{38}$Ar are the fixed concentrations of 3365 ppm and 652 ppm in accordance with the abundance ratio of the isotopes. These known concentrations are linked with measured values of the mass spectra obtained by the mass spectrometer. Based on this linkage, the concentration of $N_2$ was determined from the measured value of the mass spectra of $N_2$ of the gas of the measured object. Furthermore, it is possible to find the total amount of nitrogen N (ppm) in a metal sample while considering the measurement time, flow rate of the carrier gas, molecular weight of the measured object, mass of the sample, etc.

[Mathematical 1]

(Formula II-1)

Ni (ppm)=$N_2$ (measured value): term for conversion of concentration

x (3365 ppm-632 ppm)/($^{36}$Ar (measured value)-$^{38}$Ar (measured value)):

x (flow rate (L/sec) x sampling time (sec))/24.8 (L/mol): term for conversion of moles of standard state

x 28.0062 (g/mol): term for conversion of N mass number

/mass of metal sample (g): term for conversion of N content

t

Here, the input values of (formula II-1) will be explained.

The "measured value" is the detection intensity of the signal in a high resolution mass spectrometry device of the moles of the measured object. The "sampling time" means the time interval of measurement points at which a signal is fetched (measurement intervals). That is, if fetching a signal every second, the sampling time is 1 second.

The "flow rate" is the flow rate of the carrier gas flowing from the impulse furnace to the high resolution mass spectrometry device.

In the present example, for the $N_2$ (measured value), the detected intensity for each signal fetched was used. For the $^{36}$Ar (measured value) and $^{38}$Ar (measured value), the average values of the detection intensities of $^{36}$Ar and $^{38}$Ar measured for 5 seconds before the signal derived from $N_2$ was detected were used. These were entered into (formula II-1). The Ni (ppm) calculated by (formula II-1) for each signal fetched were cumulatively added over the time band in which a signal derived from nitrogen is detected. The total sum was found as the total amount of nitrogen N (ppm) in the ferrous sample.

Due to this technique, in the same way as the results of analysis by TCD using the conventionally essential He gas, it was learned that the nitrogen concentration in a sample can be determined by high resolution mass spectrometry using Ar. The results of quantitative determination of the JSS2 sample are shown in Table 7. Further, quantitative determination became possible by measurement of the isotope argon in the carrier without going through the conventionally necessary step of preparation of a calibration curve, so it was confirmed that various costs of the standard gas required for quantitative determination (materials, step of gas replacement, time of measurement as a whole, etc.) could be cut.

[Table 7]

Table 7. Comparison of Prior Art (TCD) and Invention Examples (MS)

| Device | Horiba EMGA930 | | Device | Horiba EMGA930 | |
|---|---|---|---|---|---|
| Sample | JSS 366-9 (N: 6.5 ppm) | | Sample | JSS 367-9 (N: 44 ppm) | |
| Measured object | N | | Measured object | N | |
| Analysis method | TCD | MS | Analysis method | TCD | MS |
| Carrier gas | He | Ar | Carrier gas | He | Ar |
| #1 | 6.6 | 6.9 | #1 | 43.5 | 44.9 |
| #2 | 7.0 | 7.4 | #2 | 43.6 | 44.9 |
| #3 | 6.4 | 6.8 | #3 | 44.4 | 42.6 |
| #4 | 7.1 | 6.0 | #4 | 45.2 | 40.9 |
| #5 | 6.5 | 5.3 | #5 | 44.7 | 39.9 |
| #6 | 7.1 | 5.9 | #6 | 44.9 | 45.2 |
| #7 | 6.4 | 6.9 | #7 | 45.6 | 45.5 |
| #8 | 6.7 | 6.1 | #8 | 44.9 | 42.5 |
| #9 | 6.8 | 7.6 | #9 | 44.0 | 41.4 |
| #10 | 7.2 | 6.1 | #10 | 45.4 | 42.0 |
| Ave (average value) | 6.8 | 6.5 | Ave (average value) | 44.6 | 43.0 |
| σ (standard deviation) | 0.3 | 0.7 | σ (standard deviation) | 0.7 | 1.9 |
| RSD (relative standard deviation) | 4.6 | 11.1 | RSD (relative standard deviation) | 1.6 | 4.7 |

(Example II-2) Results in case of quantitative determination of all measurement points by applying (formula II-1).

A test device similar to Example II-1 (FIG. 5) was used for the following analysis. Three levels of standard samples of ferrous metal made by Seishin Trading Co., Ltd., two levels of standard samples of ferrous metal of JSS, and a blank were respectively measured N=10 times.

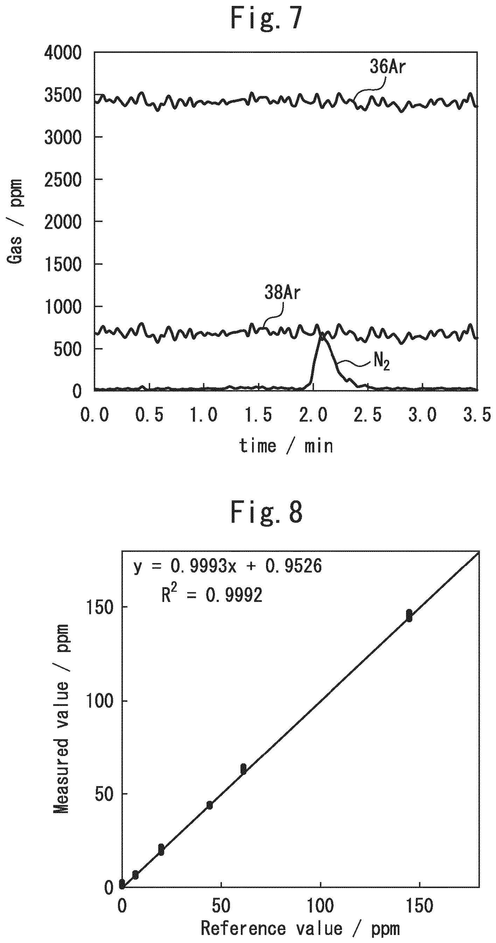

In the present example, at all of the measurement points stretching over the time band where a signal derived from nitrogen is detected, the measured $N_2$ (measured value), $^{36}$ Ar (measured value), and $^{38}$ Ar (measured value) were used to calculate Ni (ppm) by (formula II-1) . These were cumulatively added over all measurement points. The total sum was found as the total amount of nitrogen N (ppm) in the ferrous metal sample. The data obtained in the present procedure has the timing at which the sample was charged into the impulse furnace as the start point of measurement. After that, the sample was heated in the impulse furnace and $N_2$ started to be detected. The detection of $N_2$ wound down in about 30 seconds. The point at which the furnace body finished being cooled after 1 minute later was made the end point of measurement. During the 200 seconds from the start point of measurement to the end point of measurement, every 1/3 second sampling time, data of the measured value by the high resolution mass spectrometry device was recorded and is comprised of 600 data points. The total sum of the 600 points of Ni (ppm) during this time was found as the total amount of nitrogen N (ppm) in the ferrous metal sample determined in the present measurement. FIG. 7 is a view showing an example of an $N_2$ (measured value), $^{36}$ Ar (measured value), and $^{38}$ Ar (measured value) by a high resolution mass spectrometry device. In FIG. 7, about 1.5 minutes on the abscissa (time) is the start point of measurement while about 3.5 minutes is the end point of measurement.

The results of measurement of the total amount of nitrogen N (ppm) in a ferrous sample obtained by the above procedure are shown in FIG. 8. The abscissa shows the standard values of the measurement samples while the ordinate shows the quantitatively determined values. In this measurement range, it was confirmed that the chart of the measurement results exhibits high linearity, i.e., quantitative determination having a high accuracy and reproducibility is possible.

Further, in Example II-2, it was confirmed that the measurement precision is improved further from Example II-1, i.e., the standard deviation (variation) of the measured values became smaller. Table 8 shows the results of measurement by Example II-1 and the results of measurement by Example II-2 for the JSS sample side by side.

[Table 8]

Table 8. Comparison of Results of Quantitative Determination of Amount of Nitrogen in Ferrous Sample According to Invention Examples

| Device | Horiba EMGA930 | | Device | Horiba EMGA930 | |
|---|---|---|---|---|---|
| Sample | JSS 366-9 (N: 6.5 ppm) | | Sample | JSS 367-9 (N: 44 ppm) | |
| | Example II-1 | Example II-2 | | Example II-1 | Example II-2 |
| #1 | 6.9 | 6.2 | #1 | 44.9 | 44.2 |
| #2 | 7.4 | 6.9 | #2 | 44.9 | 44.5 |
| #3 | 6.8 | 6.1 | #3 | 42.6 | 43.5 |
| #4 | 6.0 | 6.4 | #4 | 40.9 | 43.4 |
| #5 | 5.3 | 6.1 | #5 | 39.9 | 43.0 |
| #6 | 5.9 | 6.1 | #6 | 45.2 | 42.9 |
| #7 | 6.9 | 6.5 | #7 | 45.5 | 43.6 |
| #8 | 6.1 | 5.7 | #8 | 42.5 | 42.9 |
| #9 | 7.6 | 6.9 | #9 | 41.4 | 42.0 |
| #10 | 6.1 | 6.4 | #10 | 42.0 | 43.4 |
| Ave (average value) | 6.5 | 6.3 | Ave (average value) | 43.0 | 43.4 |
| σ (standard deviation) | 0.7 | 0.4 | σ (standard deviation) | 1.9 | 0.6 |
| RSD (relative standard deviation) | 11.1 | 5.9 | RSD (relative standard deviation) | 4.7 | 1.4 |

In each of Example II-1 and Example II-2, the total amount of nitrogen N (ppm) in the ferrous sample was found based on (formula II-1), but in Example II-2, (formula II-1) was applied to all measurement points, whereby it was confirmed that the measurement precision was further improved and the standard deviation (variation) of the measured values became remarkably smaller.

(Example II-3)

For the quantitative determination of the nitrogen in Ti, a titanium standard sample Ti Alloy-64-001 (certified value N: 42 ppm) made by Seishin Trading Co., Ltd. was analyzed. The results of quantitative determination by the inert gas melting-thermal conductivity method using helium gas described in JIS H1612: 1993 and the results of quantitative determination by a procedure similar to Example II-2 (Example II-3) were compared. It should be noted that, for just heating of the titanium sample, as shown in JIS H 1612: 1993, heating of a titanium sample using 1 g of platinum as a bath metal was performed.

The N quantitatively determined values of a sample of a mass of 0.3 g obtained by the method of the present invention using the method based on Example II-2 and the inert gas melting-thermal conductivity method (TCD reference example) using He are shown in Table 9.

[Table 9]

| Table 9. Comparison of Results of Quantitative Determination of Nitrogen in Titanium Sample According to Invention Examples (Number of Times of Measurement N=10) | | |
|---|---|---|
| Times of measurement | TCD (Reference example) | MS (Example II-3) |
| #1 | 41.7 | 42.0 |
| #2 | 42.4 | 42.2 |
| #3 | 42.1 | 42.5 |
| #4 | 41.1 | 41.6 |
| #5 | 41.2 | 42.3 |
| #6 | 41.8 | 40.9 |
| #7 | 42.0 | 41.6 |
| #8 | 42.3 | 41.5 |
| #9 | 41.8 | 42.3 |
| #10 | 42.3 | 42.2 |
| Ave (average value) | 41.9 | 41.9 |
| $\sigma$ (standard deviation) | 0.4 | 0.5 |
| RSD (relative standard deviation) | 1.0 | 1.2 |

From the above results, it was confirmed that analysis with a high precision is possible by the present technique even in the case of a titanium sample with a relatively high melting point.

(Example II-4)

For each of six reduced iron samples, the N in the sample was quantitively determined by the following four methods:

(Method 1: Thermal Conductivity Method)
The N in the sample was quantitatively determined by the inert gas melting-thermal conductivity method using helium gas shown in JIS G 1228.
(Method 2: Mass Spectrometry Method)
The N in the sample was quantitatively determined using a modified device obtained by directly connecting an EMGA-930 made by Horiba Ltd. and a multiturn type high resolution mass spectrometer made by Kanomax Japan Ltd. An impulse furnace was used to melt the reduced iron sample in an argon gas atmosphere, the gas generated was introduced as a carrier gas to the mass spectrometer, and a method similar to Example II-2 was used to quantitatively determine the N based on the Ar isotope concentration.
(Method 3: Developed Method)
A chromatography function part was added to the above configuration of the Method 2. Specifically, gas generated at an impulse furnace was run through a packed column Shincarbon-ST of about 1 m able to separate CO and $N_2$ at room temperature, then was introduced into the mass spectrometer to quantitatively determine the nitrogen in the reduced iron.
(Method 4: Wet Analysis)
The JIS G 1228 ammonia distillation separation amide sulfate titrimetric method was used to quantitatively determine the nitrogen. This was made a reference value.

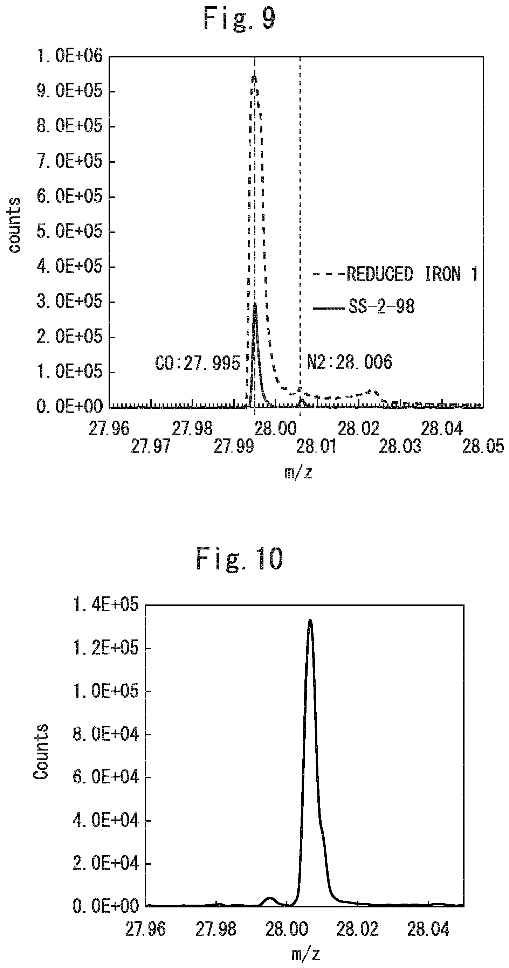

An example of the mass spectrum obtained by the method of combining an impulse furnace and mass spectrometer and using argon as a carrier gas is shown in FIG. 9. It was learned that if a standard sample of steel, even under conditions where CO and $N_2$ are sufficiently separated, if using reduced iron containing a large amount of oxygen as a sample, the tailing of peak of CO interfere with $N_2$ and the quantitatively determined value is affected.
From the peak of $N_2$, the tailing of peak of the CO are removed as background and the quantitatively determined values of nitrogen at the reduced irons were calculated. This is shown in Table 10 as the results of the mass spectrometry method of Method 2.

[Table 10]

| Table 10. Quantitative Values of Nitrogen in Samples by Different Test Methods (N_ ppm) | | | | |
|---|---|---|---|---|
| Sample | Method 1 Thermal conductivity | Method 2 Mass spectrometry | Method 3 Developed method | Method 4 Wet analysis |
| 1 | 76 | 107 | 54 | 54 |
| 2 | 44 | 39 | 28 | 30 |
| 3 | 35 | 34 | 24 | 22 |
| 4 | 66 | 39 | 36 | 37 |
| 5 | 56 | 46 | 38 | 31 |
| 6 | 64 | 56 | 42 | 50 |

The developed method of Method 3 will be explained. It was confirmed that if introducing a gas which had been passed through the column into a mass spectrometer, the time periods of appearance of the mass peaks of CO and $N_2$ change (chromatograms). From the confirmed results, the time band in which N can reliably be separated alone was selected. FIG. 10 shows one example of a mass spectrometry spectrum in that time band. A mass spectrometry spectrum of N alone can be formed. Integration was performed based on the signal intensity of $N_2$ in this time band and the quantitatively determined value at the reduced iron was calculated. This is shown in Table 10 as the results of the mass spectrometry method of the Method 3 (Developed Method))

Table 10 shows the results of quantitative determination by the thermal conductivity method of the Method 1 and the wet analysis of the Method 4. From the comparison of these results, it was confirmed that with the thermal conductivity method of the Method 1, sometimes nitrogen is excessively estimated over the wet analysis method of the Method 4, but with the developed method of the Method 3, results substantially matching the wet analysis method of the Method 4 are obtained.

**Claims**

1.  A quantitative analysis method of a measured object using a mass spectrometer, which quantitative analysis method comprising
    a gas introduction step of mixing a gas of the measured object and a carrier gas different from the gas of the measured object and having two or more natural isotopes and introducing them to a mass spectrometer,

    a mass spectrometry step of mass spectrometry of the gas of the measured object and the two or more natural isotopes in the carrier gas at the mass spectrometer, and
    a gas concentration calculation step of calculating a concentration of the gas of the measured object using analyzed values of the two or more isotopes of the carrier gas obtained by the mass spectrometry step as calibration standards.

2.  The quantitative analysis method according to claim 1, wherein the carrier gas contains at least one of Ar, $N_2$, and $O_2$ or a mixture of the same.

3.  The quantitative analysis method according to claim 1, further comprising an extraction step of heating a sample containing the measured object to make it melt in an atmosphere of the carrier gas and extracting the gas of the measured object from the sample.

4.  The quantitative analysis method according to claim 3, wherein the sample contains metal in 80 mass% or more.

5.  The quantitative analysis method according to claim 1, further comprising a gas quantity calculation step of multiplying a concentration of the gas of the measured object and a flow rate of the carrier gas flowing in the time period in which the concentration was detected so as to calculate the amount of the gas of the measured object.

6.  The quantitative analysis method according to claim 5, further comprising, at the gas quantity calculation step, subdividing the time period in which the concentration of the gas of the measured object was detected, calculating the quantity $C_i$ (i=1, 2,... n, where "n" is the number of subdivided sections) for each subdivided section, and calculating the

20

total sum of Ci as the amount of the gas of the measured object.

7. The quantitative analysis method according to claim 1, wherein the two or more natural isotopes in the carrier gas are $^{36}$Ar and $^{38}$Ar.

8. The quantitative analysis method according to claim 1, wherein the measured object is one or more of any of nitrogen, carbon, oxygen, and hydrogen.

9. The quantitative analysis method according to claim 1, wherein the mass spectrometer has a resolution enabling separation and detection of mass number 28.006 of nitrogen gas and mass number 27.995 of carbon monoxide gas.

10. The quantitative analysis method according to claim 1, further comprising analyzing the mass of the gas of the measured object with a known concentration and the carrier gas in advance by the mass spectrometer to find a correction coefficient used for calculation of the concentration of the gas of the measured object .

11. A quantitative analysis device comprising

a gas introduction function part for mixing a gas of a measured object and a carrier gas different from the gas of the measured object and having two or more natural isotopes and introducing them into a mass spectrometer,
a mass spectrometry function part provided at the mass spectrometer for mass spectrometry of the gas of the measured object and the two or more natural isotopes of the carrier gas, and
a gas concentration calculation function part for calculating a concentration of the gas of the measured object using as calibration standards the analysis values of the two or more natural isotopes of the carrier gas obtained at the mass spectrometry function part.

12. The quantitative analysis device according to claim 11, further comprising a vaporization function part heating a sample containing the measured object to make it melt in an atmosphere of the carrier gas and render the measured object a gas from the sample.

13. The quantitative analysis device according to claim 11, further comprising a gas concentration calculation function part using a signal intensity of the measured object measured by the mass spectrometry function part and the signal intensities of the two or more natural isotopes in the carrier gas to calculate the concentration of the gas of the measured object.

14. The quantitative analysis device according to claim 11, wherein the mass spectrometry function part has a resolution enabling separation and detection of the mass number 28.006 of nitrogen gas and the mass number 27.995 of carbon monoxide gas.

15. The quantitative analysis device according to claim 11, further comprising a chromatography function part able to separate the gas of the measured object and other gases.

# Fig. 1

PRIOR ART METHOD

**Day x**

counts

ppm

**Day y**

counts

ppm

**Day z**

counts

ppm

○ PLOT OF STANDARD GAS
— CALIBRATION CURVE FOUND FROM STANDARD GAS
◇ MEASUREMENT DATA

· NECESSARY TO SWITCH STANDARD GAS AND SUPPLIED GAS TO PREPARE CALIBRATION CURVE
(BY ○ POINTS) AND DETERMINE MEASURED VALUES OF ◇ EACH TIME

· NOT POSSIBLE TO COUNTER AN FLUCTUATION AFTER PREPARING CALIBRATION CURV

Fig. 2

# Fig. 3

INVENTION EXAMPLE

$^{36}$Ar 3365 ppm

$^{38}$Ar 632 ppm

-- MEASURED VALUES OF Ar ISOTOPES
◇ MEASUREMENT DATA

· ISOTOPES IN SUPPLIED GAS USED FOR CALIBRATION, SO NO NEED TO
SWITCH GASESAND MEASUREMENT POSSIBLE AT ALL TIMES

· CALIBRATION POSSIBLE AT ALL TIMES, SO HIGHER PRECISION DETERMINATION
NOT AFFECTED BY FLUCTUATIONS IN DEVICE POSSIBLE

EP 4 560 310 A1

# Fig. 4

# Fig. 5

GENERATION

CO | N_2 | H_2

Ar

IMPULSE FURNACE
O, N, H VAPORIZATION

DUST FILTER — PRESSURE REGULATOR — HIGH RESOLUTION MASS SPECTROMETER — FLOWMETER

# Fig. 6

CO
(27.9949)

N_2
(28.0062)

INTENSITY(a. u.)

MASS-TO-CHARGE RATIO m/z

# Fig. 7

# Fig. 8

# Fig. 9

# Fig. 10

Fig. 11

## INTERNATIONAL SEARCH REPORT

| International application No. |
|---|
| **PCT/JP2023/026460** |

| A. | CLASSIFICATION OF SUBJECT MATTER |
|---|---|

*G01N 27/62*(2021.01)i
FI:   G01N27/62 F; G01N27/62 C

According to International Patent Classification (IPC) or to both national classification and IPC

| B. | FIELDS SEARCHED |
|---|---|

Minimum documentation searched (classification system followed by classification symbols)

G01N27/60-27/70; G01N30/00-30/96; H01J49/00-49/48

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2006/0151695 A1 (ROTTMANN, Lothar et al.) 13 July 2006 (2006-07-13) paragraphs [0006], [0013], [0014], [0042] | 1-15 |
| Y | JP 2014-235088 A (KOKURITSU IYAKUHIN SHOKUHIN EISEI KENKYUSHO) 15 December 2014 (2014-12-15) paragraph [0009] | 1-15 |
| Y | JP 2000-65797 A (SHIMADZU CORP.) 03 March 2000 (2000-03-03) paragraph [0008] | 1-15 |
| Y | JP 3-21867 A (DAIDO STEEL CO., LTD.) 30 January 1991 (1991-01-30) p. 2, upper left column, line 1 to upper right column, line 2, lower left column, lines 9-17 | 3-4, 8, 12 |
| Y | WO 2019/225573 A1 (HORIBA, LTD.) 28 November 2019 (2019-11-28) paragraphs [0025], [0048]-[0050] | 3-4, 8, 12 |
| A | US 5051557 A (SATZGER, R, Duane) 24 September 1991 (1991-09-24) | 1-15 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 September 2023** | **26 September 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/026460**

| Patent document cited in search report | | | Publication date (day/month/year) | Patent family member(s) | Publication date (day/month/year) |
|---|---|---|---|---|---|
| US | 2006/0151695 | A1 | 13 July 2006 | (Family: none) | |
| JP | 2014-235088 | A | 15 December 2014 | (Family: none) | |
| JP | 2000-65797 | A | 03 March 2000 | (Family: none) | |
| JP | 3-21867 | A | 30 January 1991 | (Family: none) | |
| WO | 2019/225573 | A1 | 28 November 2019 | EP 3786636 A1 paragraphs [0025], [0048]-[0050]<br>US 2021/0318267 A1<br>CN 112166321 A | |
| US | 5051557 | A | 24 September 1991 | (Family: none) | |

Form PCT/ISA/210 (patent family annex) (January 2015)

**REFERENCES CITED IN THE DESCRIPTION**

**Patent documents cited in the description**

- JP 2017215275 A
- JP 2014235088 A
- JP 2000065797 A
- JP 5846344 B

**Non-patent literature cited in the description**

- *JIS G*, 2006, vol. 1228